# EUROPEAN PATENT SPECIFICATION

(11) **EP 3 394 271 B1**
(45) Date of publication and mention of the grant of the patent: **20.05.2020**
(21) Application number: 16819072.6
(22) Date of filing: 20.12.2016
(51) Int. Cl.: C12N 15/867

(54) **STABLE INTEGRATION OF SIN LENTIVIRAL TRANSFER VECTORS**
STABILE INTEGRATION VON SIN-LENTIVIRALEN TRANSFER-VEKTOREN
INTÉGRATION STABLE DE VECTEURS LENTIVIRAUX SIN DE TRANSFERT

(30) Priority: 21.12.2015 EP 15201510
(43) Date of publication of application: 31.10.2018
(73) Proprietor: MolMed SpA, 20132 Milan (IT)
(72) Inventor: STORNAIUOLO, Anna, 20132 Milan (IT); BOVOLENTA, Chiara, 20132 Milan (IT); MAVILIO, Fulvio, 91002 Evry (FR); RIZZARDI, Gian Paolo, 20132 Milan (IT)
(74) Representative: Serino, Loredana
(86) International application number: PCT/EP2016/081964
(87) International publication number: WO 2017/108817

(56) References cited:
- WO-A2-2010/022195
- F. DI NUNZIO ET AL: "Transduction of Human Hematopoietic Stem Cells by Lentiviral Vectors Pseudotyped with the RD114-TR Chimeric Envelope Glycoprotein", HUMAN GENE THERAPY, vol. 18, no. 9, 1 September 2007 (2007-09-01), pages 811-820, XP55013461, ISSN: 1043-0342, DOI: 10.1089/hum.2006.138
- LIEBER A ET AL: "Integrating adenovirus-adeno-associated virus hybrid vectors devoid of all viral genes.", JOURNAL OF VIROLOGY NOV 1999, vol. 73, no. 11, November 1999 (1999-11), pages 9314-9324, XP002769327, ISSN: 0022-538X

## Description

### Field of the invention

The present invention relates to the field of the production of lentiviral vectors (LV) for gene therapy. More particularly, the invention relates to a system to obtain the stable integration of a Self Inactivating (SIN) lentiviral transfer vector into a packaging cell line. The SIN lentiviral transfer vector is integrated using a DNA fragment containing the Inverted Terminal Repeats (ITR) of Adeno Associated Virus (AAV), thus obtaining the stable producer cell line for SIN lentiviral vectors.

### Background

More than a decade of research and development has drastically enriched the efficacy and safety of HIV-based lentiviral vector (LV) technology in gene therapy. The implementation of stable LV packaging cells in clinic represents a mandatory milestone to reduce the manufacturing cost and to enhance the overall quality and safety of the vectors. Thus far, several strategies have generated stable LV packaging cells differing, essentially, for the delivery of the packaging genes, i.e. plasmids vs integrating vectors, and different nature of the pseudo-typing envelope. Once obtained a stable packaging cell line including lentiviral structural and regulatory proteins, as well as the preferred envelope protein, it is necessary to integrate the transfer vector, incorporating the gene of interest, into the genome of the cell line. The resulting stable cell line is called producer cell line. The producer cell line is the definitive stable tool for the production of lentiviral vectors including the gene of interest.

While several recombinant systems exist that, in principle, allow the stable integration of foreign genes into the genome of target cells, in case of complex genes or expression systems, it is necessary to construct an appropriate gene delivery vehicle that allow the integration, as well as the functionality of the final expression cassette. This is particularly important in respect to the development of producer cell lines for Self-Inactivating Lentiviral Vectors (SIN). In this kind of vectors, the deletion of lentiviral enhancer and promoter sequences from the 3' LTR results in the generation of vectors which, on infection of target cells, are incapable of transcribing vector-length RNA. Because of this modification, integrated SIN vectors are incapable of further replication thus reducing the likelihood of generating replication-competent viruses as well as the danger of inadvertently influencing transcription activity of nearby endogenous promoters. Because of its structure, the introduction of SIN transfer vectors into packaging cell lines cannot be accomplished with the traditional method of viral transduction that, by virtue of the inactivating deletion results to be incompatible with the integration of a functional transfer vector.

To overcome this problem, Xu et al. 2001 developed a conditional SIN transfer vector, in which they replaced the 3' LTR U3 transcription regulatory elements with an inducible promoter (Tet-responsive element). This kind of systems may solve the problem, but they are a compromise in the vector design, since the LTR may retain a basal level of expression.

One of the possible way to obtain stable integration of a fully SIN transfer vector by non retroviral method is the cotransfection of a vector plasmid and a selectable drug resistance plasmid (Ikeda et al. 2003). This system results to be not efficient for both integration and stability of integrates. Throm et al. 2009 disclose a producer cell line in which a fully SIN transfer vector is stably integrated into the genome by concatemeric array transfection. Such array is obtained through the ligation of DNA fragments encoding the SIN transfer vector genome, with drug resistance cassettes included into the array. Even though the disclosed method results to be effective for the development of a producer cell line, the system include the further step of array formation, thus adding further level of complexity to the technology.

The prior art also includes Di Nunzio et al, HUMAN GENE THERAPY, vol. 18, no. 9, 1 2007, 811-820, disclosing the pseudotyping of lentiviral vectors with the RD114-TR glycoprotein (and being applied to a SIN-GFP transfer vector).

There is a need to develop simpler methods to integrate full SIN transfer vector genome into a stable packaging cell line, in order to obtain an effective producer cell line. The present invention addresses this need.

### Summary of the invention

The present invention is related to the field of production of Self Inactivating Lentiviral Vectors (SIN LV). Several gene therapy clinical trials are ongoing employing SIN-LV as gene delivery vehicles. For reasons of safety, this kind of vectors should be regarded as vectors of choice in clinical trials. In the large majority of clinical trials, the production of SIN LV is still based on transient protocols. One of the key issue to be resolved for the development of stable producer cell line for SIN LV is the development of a simple system that allows the stable integration of SIN transfer vectors.

The present invention provides a new strategy to integrate lentiviral SIN transfer vector into the genome of packaging cell lines, which already contain structural lentiviral protein (gag/pol) the regulatory protein rev and the envelope protein of choice.

Such strategy is based on the transfection of DNA fragments comprising the Inverted Terminal Repeats (ITR) of the Adeno-Associated Virus (AAV) at the extremities 5' and 3' flanking a complex construct containing at least a SIN transfer vector and an antibiotic resistance gene, that is positioned downstream of the lentiviral 3' LTR and is expressed by an independent promoter.

By simply transfecting the DNA fragments above into a packaging cell line including lentiviral structural (gag/pol) and regulatory (rev) components and the envelope protein, it was possible to obtain a stable producer cell line able for SIN LV.

### Statements of the invention

According to a first aspect of the invention there is provided a system for stable integration of a SIN lentiviral transfer vector into a packaging cell line, wherein such a system consists of a DNA fragment comprising the following elements, starting from the 5' end:
i. The Inverted Terminal Repeat of the Adeno Associated Virus (AAV ITR)
ii. A SIN lentiviral transfer vector
iii. A constitutive promoter that regulates the expression of an antibiotic resistance gene
iv. An antibiotic resistance gene
v. The Inverted Terminal Repeat of the Adeno Associated Virus (AAV ITR)

In one embodiment the SIN lentiviral transfer vector comprises at position 5' the entire lentiviral Long Terminal Repeat (LTR) and, at position 3', a deletion of the enhancer and promoter sequences of the lentiviral LTR.

In another embodiment the SIN lentiviral transfer vector comprises at position 5' a lentiviral LTR in which the U3 segment has been substituted by a promoter and, at position 3', a deletion of the enhancer and promoter sequences of the lentiviral LTR. The substituted promoter is selected from CMV, CMV IE, PGK, SV40, eF1α SFFV and RSV more preferably the promoter is a CMV IE promoter.

The antibiotic resistance gene employed in the system is selected from hygromycin, kanamycin, neomycin, bleomycin, phleomycin or Zeocin® resistance gene, preferably the Zeocin® resistance gene. A promoter selected from CMV, CMV IE, PGK, SV40, eF1α, SFFV and RSV, regulates the expression of the resistance gene preferably the promoter is a SV40 promoter.

In another aspect of the present invention there is provided a method to obtain a producer cell line for SIN lentiviral vectors comprising:
1) Preparing a DNA fragment comprising, starting from the 5' end:
   i. The Inverted Terminal Repeat of the Adeno Associated Virus (AAV ITR)
   ii. A SIN lentiviral transfer vector
   iii. A constitutive promoter that regulates the expression of an antibiotic resistance gene
   iv. An antibiotic resistance gene
   v. The Inverted Terminal Repeat of the Adeno Associated Virus (AAV ITR)
2) Transfecting a packaging cell line for lentiviral vectors with such DNA fragment
3) Selecting the producer cell line by culturing the cells in the presence of the antibiotic

In one embodiment the SIN lentiviral transfer vector comprises at position 5' the entire lentiviral Long Terminal Repeat (LTR) and, at position 3', a deletion of the enhancer and promoter sequences of the lentiviral LTR.

In another embodiment the SIN lentiviral transfer vector comprises at position 5' a lentiviral LTR in which the U3 segment has been substituted by a promoter and, at position 3', a deletion of the enhancer and promoter sequences of the lentiviral LTR. The substituted promoter is selected from CMV, CMV IE, PGK, SV40, eF1α SFFV and RSV, more preferably the promoter is a CMV IE promoter.

The antibiotic resistance gene employed in the system is selected from hygromycin, kanamycin, neomycin, bleomycin, phleomycin or Zeocin® resistance gene, preferably the Zeocin® resistance gene. A promoter selected from CMV, CMV IE, PGK, SV40, eF1α, SFFV and RSV, regulates the expression of the resistance gene, preferably the promoter is a SV40 promoter.

Preferably the packaging cell line is a stable packaging cell line obtained from an host cell line containing into its genome lentiviral gag/pol, lentiviral rev and an envelope protein. In a further embodiment the packaging cell line further, contains lentiviral tat stably integrated into the genome of the cell.

The host cell line is a human cell line preferably selected from HEK293, HEK293-T, HEK293-SF, TE671, HT1080 or HeLa, more preferably the cell line is HEK293-T.

The env gene is selected from VSV-G env, MLV 4070 env, RD114 env, chimeric envelope protein RD114-TR, chimeric envelope protein RD114pro, baculovirus GP64 env or GALV env or derivatives thereof, more preferably the env gene is the gene encoding the RD114-TR.

In a further aspect of the invention, there is provided the producer cell line for SIN lentiviral vectors obtainable by the method above.

In an additional aspect of the invention, there is provided a producer cell line containing into its genome at least one copy of a DNA fragment comprising, starting from the 5' end:
i. The Inverted Terminal Repeat of the Adeno Associated Virus (AAV ITR)
ii. A SIN lentiviral transfer vector
iii. A constitutive promoter that regulates the expression of an antibiotic resistance gene
iv. An antibiotic resistance gene
v. The Inverted Terminal Repeat of the Adeno Associated Virus (AAV ITR)

In another aspect of the invention, there is provided a method to produce SIN lentiviral vectors comprising culturing a producer cell line containing into its genome at least one copy of a DNA fragment comprising starting from the 5' end:
i. The Inverted Terminal Repeat of the Adeno Associated Virus (AAV ITR)
ii. A SIN lentiviral transfer vector
iii. A constitutive promoter that regulates the expression of an antibiotic resistance gene
iv. An antibiotic resistance gene
v. The Inverted Terminal Repeat of the Adeno Associated Virus (AAV ITR)

### Detailed description of the invention

A detailed description of preferred features and embodiments of the invention will be described by way of non-limiting example.

The invention can be put into practice by a person of ordinary skill in the art, who will employ, unless otherwise indicated, conventional techniques of chemistry, molecular biology, microbiology, recombinant DNA and immunology. All such techniques are disclosed and explained in published literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F.M. et al. (1995 and periodic supplements; Current Protocols in Molecular Biology, ch. 9, 13, and 16, John Wiley & Sons, New York, N.Y.); Current Protocols in Immunology, ch. 12, John Wiley & Sons, New York, N.Y.); B. Roe, J. Crabtree, and A. Kahn, 1996, DNA Isolation and Sequencing: Essential Techniques, John Wiley & Sons; J . M. Polak and James O'D. McGee, 1990, In Situ Hybridization: Principles and Practice; Oxford University Press; M. J. Gait (Editor), 1984, Oligonucleotide Synthesis: A Practical Approach, Irl Press; and, D. M. J. Lilley and J. E. Dahlberg, 1992, Methods of Enzymology: DNA Structure Part A: Synthesis and Physical Analysis of DNA Methods in Enzymology, Academic Press.

### System for integration of SIN transfer vector

The present invention provides a new strategy to obtain a stable producer cell line necessary for the production of Self Inactivated (SIN) lentiviral vectors. As used herein the term stable producer cell line means a packaging cell line for lentiviral vectors in which at least one copy of a lentiviral transfer vector has been introduced, and stably integrated into its genome.

As used herein the term packaging cell line means an host cell line containing into its genome lentiviral structural protein gag/pol, the regulatory lentiviral protein rev, and the envelope protein of interest. Preferably the envelope protein is selected from the group consisting of VSV-G env, MLV 4070 env, RD114 env, chimeric envelope protein RD114-TR, chimeric envelope protein RD114pro, baculovirus GP64 env or GALV env. Optionally the packaging cell line may further contain stably integrated into its genome the lentiviral protein tat.

The term "lentiviral transfer vector" means a construct containing the gene of interest flanked at least by the lentiviral sequences necessary for the packaging into the virions (i.e: psi signals) and for integration into the genome of the transduced cell (i.e.: 5' and 3' Long Terminal Repeats, LTR). More particularly, the lentiviral transfer vector construct is a DNA fragment including at least the following components starting from the 5'end: the lentiviral 5' LTR, the splice donor site (SD), the packaging sequence (psi, Ψ), the truncated gag sequence, and the lentiviral Rev Responsive Element (RRE) the splice acceptor site (SA), a gene of interest whose expression is regulated by an independent promoter and the lentiviral 3' LTR. Optionally additional elements may be included in the transfer vector such as a WPRE or the central polypurine tract (cPPT). Optionally, the 5' LTR of the transfer vector may be modified in such a way that the U3 region of the 5' LTR may be substituted by a constitutive promoter selected from CMV, CMV IE, PGK, SV40, eF1α SFFV and RSV so as to obtain a third generation lentiviral vector.

The systems developed according to the present invention are aimed to the stable production of the so-called SIN lentiviral vectors, characterized in that they contain a 3'LTR with a deletion of the lentiviral enhancer and promoter sequences. In a particular embodiment, the SIN lentiviral vector contains a deletion of 400 nucleotides in the 3'LTR as disclosed in Zuffrey et al. 1998. Self-inactivation relies on the presence of this deletion that, during the reverse transcription, is transferred to the 5' LTR of the proviral DNA thus rendering the viral genome integrated into the transduced cell unable to be further transcribed.

The production of a SIN lentiviral vector requires the use of a transfer vector containing a deletion of lentiviral enhancer and promoter sequences in the 3' LTR. Because of this structure, the introduction of SIN transfer vectors into packaging cell lines cannot be accomplished with the traditional method of viral transduction that, by virtue off the inactivating deletion, results to be incompatible with the integration of a functional transfer vector.

The present invention provides a new system for efficient integration of a SIN transfer vector into the genome of a packaging cell line in order to obtain a stable producer cell line. The system consists of a DNA fragment comprising, starting from the 5' end:
i. The Inverted Terminal Repeat of the Adeno Associated Virus (AAV ITR)
ii. A SIN lentiviral transfer vector
iii. A constitutive promoter that regulates the expression of an antibiotic resistance gene
iv. An antibiotic resistance gene
v. The Inverted Terminal Repeat of the Adeno Associated Virus (AAV ITR)

The DNA fragment may be linear or circular.

The DNA fragment may results from the linearization of a plasmid containing the above-mentioned components. Alternatively, the DNA fragment may contain only the sequences between the two AAV-ITRs.

The presence of the AAV ITRs allows for the stable integration into the genome of the packaging cell line. The antibiotic resistance gene is positioned out of the lentiviral LTR, particularly downstream the lentiviral 3'LTR. Thanks to this configuration, it is possible to select the cell clones in which the foreign gene is stably integrated, without incorporating the antibiotic resistance gene into the final viral vector.

In a further embodiment there is provided a method to obtain a stable producer cell line for SIN lentiviral vector comprising:
1) Preparing DNA fragments comprising starting from the 5' end, at least the following components:
   i. The Inverted Terminal Repeat of the Adeno Associated Virus (AAV ITR)
   ii. A SIN lentiviral transfer vector
   iii. A constitutive promoter that regulates the expression of an antibiotic resistance gene
   iv. An antibiotic resistance gene
   v. The Inverted Terminal Repeat of the Adeno Associated Virus (AAV ITR)
2) Transfecting a packaging cell line for lentiviral vector with such DNA fragment
3) Selecting the producer cell line by culturing the cells in the presence of the antibiotic

Several kind of packaging cell lines have been disclosed in the art, that differ from each other only for the integration system of the lentiviral structural gag/pol, the regulatory protein rev as well as the envelope protein. For example WO 04/22761 discloses packaging cell line in which lentiviral structural protein are integrated into the host cell by transduction with gamma retroviral vectors, while Throm et al. 2009 employ SIN gamma retroviral vectors. In WO 2012/028681 the integration of gag/pol and rev is obtained using of a complex construct with two independent expression cassettes incorporated in a baculo AAV hybrid vector, in WO2013121194 lentiviral gag/pol is integrated by site specific recombination. All these examples of stable packaging cell line can be used to obtain a stable producer cell line by transfecting the DNA fragments according to the present invention containing a SIN lentiviral transfer vector followed by an expression cassette with an antibiotic resistance gene, such fragment flanked by AAV ITRs.

In an additional embodiment there is provided a producer cell line for SIN lentiviral vector containing into its genome at least one copy of a DNA fragment comprising, starting from the 5' end, at least the following components:
i. The Inverted Terminal Repeat of the Adeno Associated Virus (AAV ITR)
ii. A SIN lentiviral transfer vector
iii. A constitutive promoter that regulates the expression of an antibiotic resistance gene
iv. An antibiotic resistance gene
v. The Internal Terminal Repeat of the Adeno Associated Virus (AAV ITR)

### Description of the figures

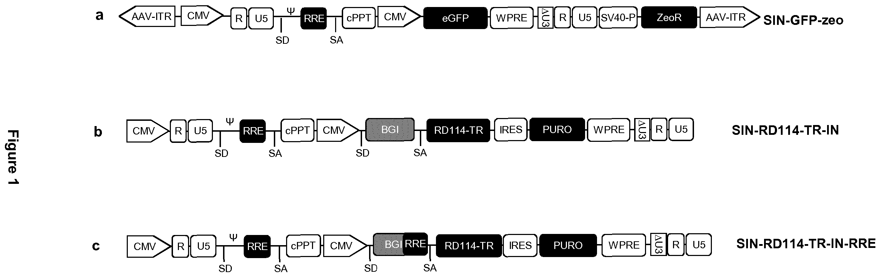

**Figure 1****. (a) Scheme of the SIN-GFP TV.** Abbreviations: AAV-ITR, Adeno-Associated Virus-Inverted Terminal Repeat; CMV, cytomegalovirus promoter; ΔU3, deleted U3, RRE, rev responsive element; SD, splice donor; SA, splice acceptor; Ψ, packaging signal; WPRE, Woodchuck Hepatitis Post-Transcriptional Regulatory Element; cPPT, central PolyPurine Tract; SV40-P, Simian Virus 40 promoter; zeoR, zeocin resistance gene. **(b, c) Schemes of the SIN-RD114-TR LVs.** Abbreviations: CMV, cytomegalovirus promoter; ΔU3, deleted U3; IN, intron; BGI, rabbit beta-globin intron, RRE, Rev Responsive Element; A, polyA sequence; IRES, internal ribosome entry site; SD, splice donor; SA, splice acceptor; Ψ, packaging signal; WPRE, woodchuck hepatitis post-transcriptional regulatory element; cPPT, central Poly-Purine Tract.

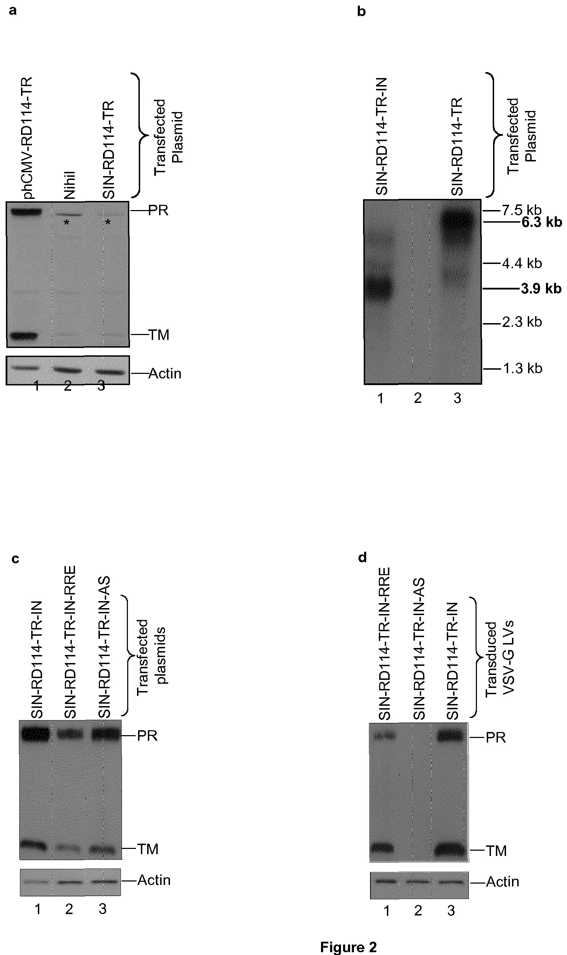

**Figure 2****.** Analysis of the vector proteins in the producer cells and in the corresponding LVs. Western blot analysis of cellular extracts (35 µg/sample) obtained from RD3-MolPack24 **(a)** and RD2-MolPack64 **(b)** producer cells and from the corresponding supernatants containing viral particles (100 ng p24Gag/sample). The membranes were hybridized sequentially with an anti-HIV human serum and the indicated anti-Rev, anti-Tat and anti-RD114-TR specific Abs and, after stripping, with the anti-actin Ab. The asterisk (*) indicates a non specific band.

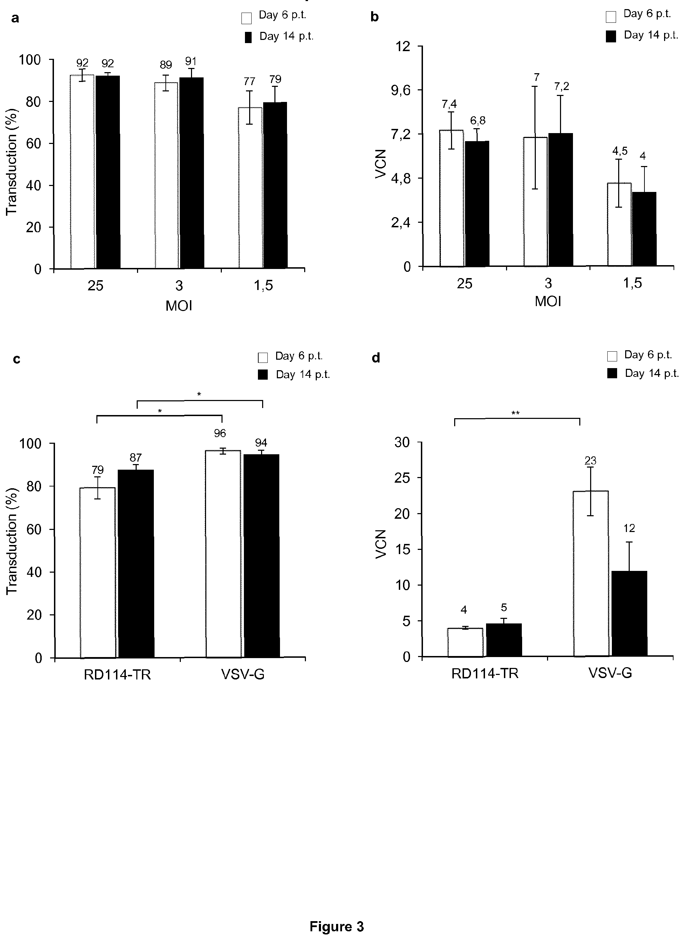

**Figure 3****.** Transduction efficiency of activated peripheral blood T lymphocytes with either RD3-MolPack24 or VSV-G pseudo-typed SIN-GFP-zeo LVs. **(a)** Total PBMC were pre-activated with CD3/CD28 Dynabeads for 48 hours and cultured in the presence of IL-7 and IL-15. T lymphocytes were then transduced with the RD3-MolPack24 LVs at the indicated MOI and six and 14 days after transduction, GFP expression was evaluated by FACS analysis to calculate transduction efficiency. **(b)** Quantification of the SIN-GFP-zeo VCN in T lymphocytes of panel a) by Q-PCR using specific primer-probe sets recognizing the packaging (Ψ) signal of the integrated vector and the genomic telomerase gene as control. **(c)** PBMC were pre-activated as in (a) and then transduced with 36ng of p24Gag equivalents of either RD3-MolPack24 LVs or VSV-G pseudo-typed LVs carrying the SIN-GFP-zeo TV. Six and 14 days after transduction, the GFP expression was evaluated by FACS analysis. **(d)** Quantification of the SIN-GFP-zeo VCN in T lymphocytes of panel c) as described in (b). Results are average ± SEM of n=3 independent experiments. * p≤ 0.05; ** p≤ 0.01.

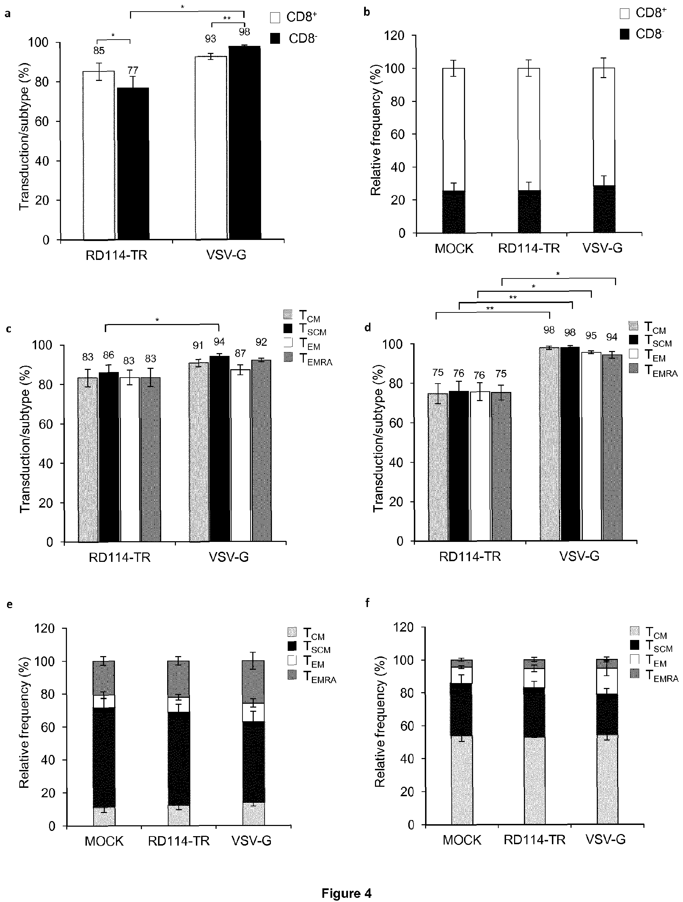

**Figure 4****.** Transduction efficiency of activated peripheral blood T lymphocyte subsets with either RD3-MolPack24 or VSV-G pseudo-typed SIN-GFP-zeo LVs. **(a)** Total peripheral blood mononuclear cells (PBMC) were pre-activated with CD3/CD28 Dynabeads for 48 hours and cultured in the presence of IL-7 and IL-15. T lymphocytes were then transduced with 36-ng p24Gag equivalent of RD3-MolPack24 LVs and six days after transduction, GFP expression was evaluated in CD3+/CD8+ and CD3+/CD8- T cell subsets **(a),** and in T cell memory subpopulations of CD3+/CD8+ and CD3+/CD8- T cell subsets **(c)** and **(d)** by FACS analysis. **(b)** Relative frequency of CD3+/CD8+ and CD3+/CD8- T cell subsets in LV- and mock-transduced cells. **(e** and **f)** Relative frequency of T central memory (TCM), T Stem Cell Memory (TSCM), T Effector Memory (TEM) and T Effector Memory RA+ (TEMRA) in either CD3+/CD8+ **(e)** or CD3+/CD8- T cell subsets **(f)** of LV- and mock-transduced cells. Results are mean ± SEM of n=4 independent experiments (day 6 p.t.). * p≤ 0.05; ** p≤ 0.01.

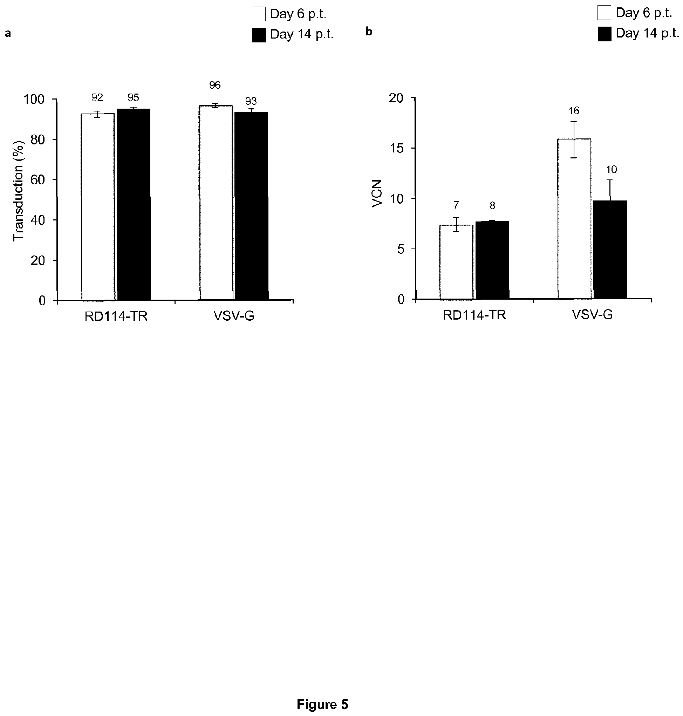

**Figure 5****.** Transduction efficiency of activated PB T lymphocytes with either RD3-MolPack24 or VSV-G pseudo-typed SIN-GFP-zeo LVs at MOI 25. **(a)** Total PBMC were pre-activated with CD3/CD28 Dynabeads for 48 hours and cultured in the presence of IL-7 and IL-15. T lymphocytes were then transduced with the RD3-MolPack24 LV s VSV-G pseudo-typed LVs carrying the SIN-GFP-zeo TV at MOI 25 and six and 14 days after transduction, GFP expression was evaluated by FACS analysis to calculate transduction efficiency. **(b)** Quantification of the SIN-GFP-zeo VCN in T lymphocytes of panel a) by Q-PCR using specific primer-probe sets recognizing the packaging (Ψ) signal of the integrated vector and the genomic telomerase gene as control. Results are average ± SEM of n=3 independent experiments.

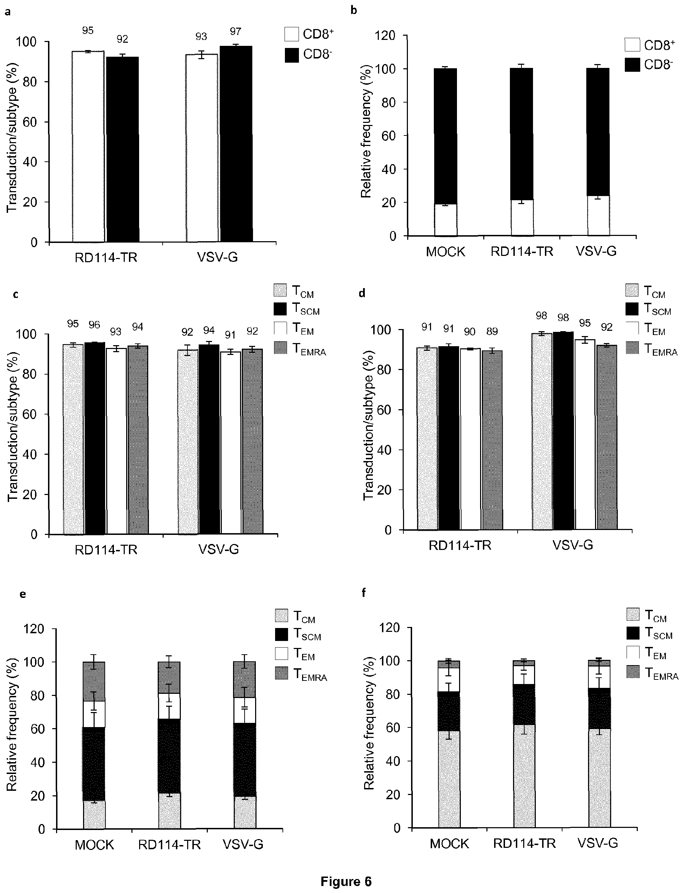

**Figure 6****.** Transduction efficiency of activated PB T lymphocyte subsets with either RD3-MolPack24 or VSV-G pseudo-typed SIN-GFP-zeo LVs at MOI 25. **(a)** Total PBMC were pre-activated with CD3/CD28 Dynabeads for 48 hours and cultured in the presence of IL-7 and IL-15. T lymphocytes were then transduced with the RD3-MolPack24 LVs at MOI 25 and six days after transduction, GFP expression was evaluated in CD3+/CD8+ and CD3+/CD8- T cell subsets **(a),** and in T cell memory subpopulations of CD3+/CD8+ and CD3+/CD8- T cell subsets (c) and **(d)** by FACS analysis. (b) Relative frequency of CD3+/CD8+ and CD3+/CD8- T cell subsets in LV- and mock-transduced cells. **(e** and **f)** Relative frequency of T Central Memory (TCM), T Stem Cell Memory (TSCM), T Effector Memory (TEM) and T Effector Memory RA+ (TEMRA) in either CD3+/CD8+ **(e)** or CD3+/CD8- T cell subsets **(f)** of LV- and mock-transduced cells. Results are average ± SEM of n=3 independent experiments.

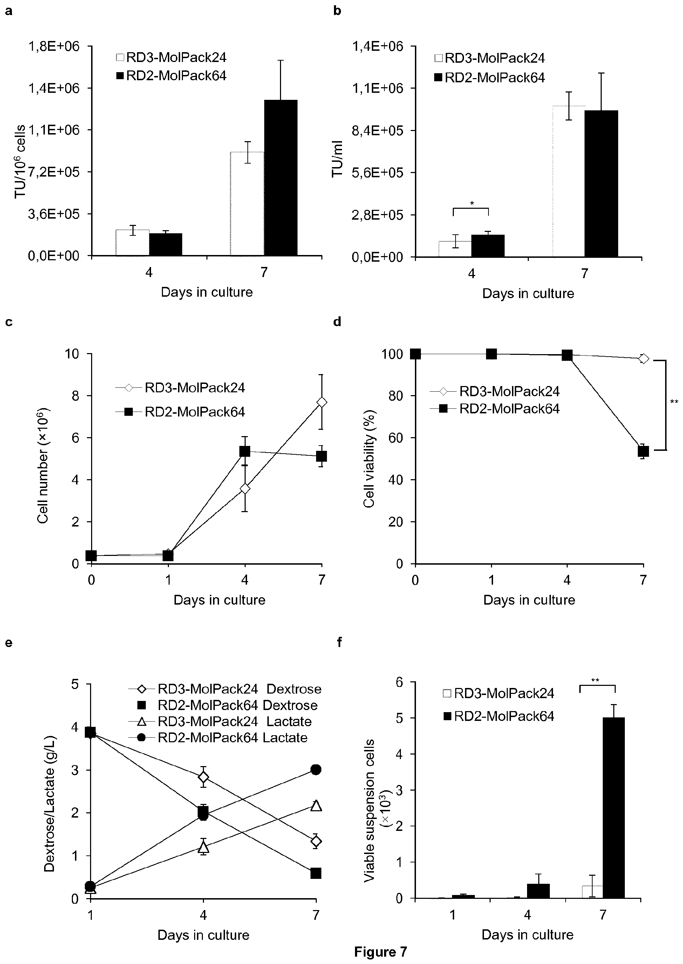

**Figure 7****.** Quantification of LV production and cell metabolism of RD3-MolPack24 and RD2-MolPack64 cells. **(a)** Analysis of the LV production normalized per number of cell and **(b)** per volume after 4 and 7 days of culture in standard T25 flasks. **(c)** Cell number and **(d)** viability at the indicated time points. **(e)** Dextrose consumption and lactate production at the indicated time points. **(f)** Viable cells in suspension at the indicated time points. **p≤0.01.

### Examples

### Example I: Derivation of several RD3-MolPack-SIN-GFP producer cells

The producer cell line RD3-MolPack-SIN-GFP were obtained starting from the packaging cell line RD-MolPack disclosed in WO2012028681. Briefly RD-Molpack packaging cell line were obtained by serially loading traced HEK-293T cells with all vector genes: first HIV-1 gag, pol, rev and hygro-resistance genes by a chimeric baculo-AAV vector, thereby obtaining the so-called PK-7 clone. The rd114-tr gene was later introduced in PK-7 cells by SIN-LV delivery. Particularly, in order to obtain an efficient transduction of the rd114tr gene, PK-7 cells were transduced with VSV-G pseudo-typed transiently derived LVs containing the SIN-RD114-TR-IN and SIN-RD114-TR-IN-RRE transfer vectors (Fig. 1b and c), to generate different RD3-MolPack packaging cells. After puromycin selection for a couple of weeks and measurement of the SIN-RD114-TR vector copy number (VCN) in several colonies, the PK-7-RD314 and PK-7-RD28 cells, containing 6 copies of the SIN-RD114-TR-IN and 12 copies of the SIN-RD114-TR-IN-RRE, respectively, were chosen as two independent RD3-MolPack packaging cells (Table 1).

**Table 1: Vector copy number^{a} of integrated genes in RD-MolPack packaging and producer cells**

| **RD-MolPack packaging** | **Gag/Pol/Rev** | **Tat** | **RD114-TR** | **RD-MolPack producer** | **SIN-GFP-zeo** |
|---|---|---|---|---|---|
| **PK-7-RD314** (RD114-TR-IN) | 2 | n.a. | 6.00 | **RD3-MolPack1** | 15.7 |
| **PK-7-RD28** (RD114-TR-IN-RRE) | 2 | n.a. | 12.0 | **RD3-MolPack24** | 48.0 |
| **PK-7-RD28** (RD114-TR-IN-RRE) | 2 | n.a. | 12.0 | **RD3-MolPack28** | 119.0 |
| **PK-7-Tat7-RD19** (RD114-TR-IN-RRE) | 2 | 6 | 13.0 | **RD2-MolPack64** | 2.70 |

| | | | | | |
|---|---|---|---|---|---|
| ^{a}The vector copy number (VCN) was calculated by Q-PCR at least in three independent experiments using specific primers and probe sets, as reported in the supplementary material. n.a., not applicable. | | | | | |

The final step to obtain a prototypic 3rd-generation RD3-MolPack producer cells for SIN-LVs, consisted in introducing the SIN-TV into either PK-7-RD314 or PK-7-RD28 packaging cells. Contrary to 2nd-generation producers, in which the LTR-TV is integrated by transduction, in 3rd-generation cells, this method cannot be applied because by doing so the TV is self-inactivated at the 5'LTR. The SIN-TV must be therefore integrated by stable transfection. The SIN-GFP-zeo transfer vector (TV) is disclosed in Fig. 1a. The transfer vector includes a SIN GFP cassette containing a CMV promoter followed by a ΔU3 lentiviral 5'LTR, the fragment between the splice donor and SA sites including the packaging signal Ψ and the lentiviral RRE (Dull et al. 1998); the central PolyPurine Tract (cPPT) (Follenzi et al. 2000); and the Woodchuck Hepatitis Post-Transcriptional Regulatory Element (WPRE) a Self Inactivating lentiviral 3' LTR, containing a deletion of 400 nucleotides in the 3'LTR (Zuffrey et al. 1998). This construct was cloned into pBSAAVzeo plasmid, generating an intermediate SIN-GFP-zeo construct in which the TV is also flanked by two Adeno-Associated Virus (AAV)-Inverted Terminal Repeats (ITRs) (Palombo et al. 1998) and contains an SV40P-zeocin expression cassette downstream the 3'ΔU3-LTR (Fig. 1a). After Pstl cut, the SIN-GFP-zeo cassette was cloned into the into the Smal site of pGEM3.1MluI construct thereby generating the SIN-GFP-zeo TV plasmid. The SIN-GFP-zeo TV plasmid was then linearized by Pstl to generate the SIN-GFP-zeo DNA fragment which was transfected in three independent RD-MolPack packaging cells: the 3rd-generation PK-7-RD314 and PK-7-RD28 described above and the 2nd-generation PK-7-Tat7-RD19 (i.e. RD2-MolPack) cells previously described in WO2012028681. The transfection was performed using kit Promega: ProFection® Mammalian Transfection System-Calcium Phosphate #E1200. We tested also the RD2-MolPack to verify whether Tat could somehow improve the performance of the SIN-LVs. After zeocin selection and the screening of several colonies by physical and functional titer for each cell type (Table 2), four independent producer cells were picked: 1) the RD3-MolPack1, derived from the PK-7-RD314cells carrying the SIN-RD114-TR-IN and containing 16 copies of SIN-GFP-zeo; 2-3) the RD3-MolPack24 and RD3-MolPack28, both derived from the PK-7-RD28 cells, carrying the SINRD114-TR-IN-RRE and containing 48 and 119 copies of the TV, respectively; and 4) the RD2-MolPack64, derived from the PK-7-Tat7-RD19 cells, carrying the SIN-RD114-TR-IN-RRE and containing 2.7 copies of the TV (Table 1).

| **Table2: Characterization of Rd-MolPack-SIN-GFP producer cells** | | | | | | | |
|---|---|---|---|---|---|---|---|
| **RD3- MolPack packaging** | **No.** | **RD3-MolPack producer cells** | **Viability (%)a** | **Titer (TU/ml)** | **Titer (TU/cell/day)** | **p24Gag (ng/ml)** | **Infectivity (TU/ng)** |
| **PK-7-RD314** | 60b/47c | **RD3-MolPack1d** | 93 | 2.8×10⁴±9.2×10³ | 0.0056±0.002 | 13.0±6.20 | 3.8×10³±2.6×10³ |
| **PK-7-RD28** | 7b | **RD3-MolPack24e** | 90 | 3.7×10⁵±1.5×10⁵ | 0.074±0.039 | 38.3±11.1 | 8.5×10³±1.5×10³ |
| **PK-7-RD28** | 7b | **RD3-MolPack28d** | 80 | 2.2×10⁵±5.4×10⁴ | 0.043±0.011 | 17.5±3.40 | 1.2×10⁴±2.1×10³ |

| **RD2-MolPack packaging cells** | **No.** | **RD2-MolPack producer cells** | **Viability (%)a** | **Titer (TU/ml)** | **Titer (TU/cell/day)** | **p24Gag (ng/ml)** | **Infectivity (TU/ng)** |
|---|---|---|---|---|---|---|---|
| **PK-7-Tat7-RD19** | 68b | **RD2-MolPack64f** | 91 | 0×10⁴±7.5×10³ | 0.056±0.009 | 40.4±4.4 | 1.8×10³±1.4×10² |

a Viability of the cells at the time of supernatant harvest (24h after cell seeding). b Number of picked-up colonies after zeocin selection. c Number of picked-up clones after limiting dilution cloning. d Mean±SEM of n = 3. e Mean±SEM of n = 6. f Mean±SEM of n = 3.

### Characterization of the RD3-MolPack-SIN-GFP producers

The expression of RD3-MolPack24 and RD2-MolPack64 vector proteins was verified by Western blot analysis of cellular and virion extracts (Fig. 2). All proteins were properly translated in packaging cells, producer cells and properly processed in the derived viral particles (Fig. 2a and 2b).

### Transduction of activated T lymphocytes with the RD3-MolPack24

We focused our attention to the transduction of PB T lymphocytes as key cells for the treatment of hematological malignancies. We found that CD3/CD28 activated PB T lymphocytes were >92% transduced by the RD3-MolPack24 LVs at MOI = 25 and >75% at MOI = 1.5 either at 6 or 14 days after transduction (Fig. 3a). Accordingly, the VCN remained stable up to 14 days after transduction, ranging from 7.4 to 4 at MOI = 25 and MOI = 1.5, respectively (Fig. 3b).

We then compared the transduction efficiency of 36-ng p24Gag equivalents of either RD3-MolPack24 LVs (infectivity = 9 × 103 TU/ng p24Gag) or transiently derived VSV-G pseudo-typed SIN-GFP-zeo LVs (infectivity = 3 × 105 TU/ng p24Gag) on CD3/CD28 activated T cells. Six days after transduction, the percentage of GFP+ cells was almost comparable (Fig. 3c), both in CD3+/CD8- and CD3+/CD8+ subsets (Fig. 4a). In contrast, at day 14, the VCN of the GFP transgene in T cells transduced with the RD114-TR pseudo-typed LVs was three fold lower than in T cells transduced with the VSV-G pseudo-typed LVs (Fig. 3d). Most importantly, T cell memory subpopulations of the CD3+/CD8- and CD3+/CD8+ subsets were transduced with identical efficiency by the two differently pseudotyped LVs (Fig. 4c and Fig. 4d). Yet, transduction with both LVs did not affect the T-cell differentiation phenotype, which was similar to mock-transduced cells (Fig. 4b, Fig. 4e and Fig. 4f). Analogous findings were observed when the two LVs were used at the same MOI (Fig. 5 and 6).

### Cell metabolism of RD3-MolPack24 and RD2-MolPack64 cells

To investigate the potential manufacturing scalability of RD2- and RD3-MolPack technology, we seeded both RD2-MolPack64 and RD3-MolPack24 cells at 1.5 × 104/cm2 in standard flask and monitored several parameters for 7 days of continuous culture (Fig. 7). Interestingly, while the two systems were comparable in terms of LV production when evaluated either as TU/cell or 10 TU/ml (Fig. 7a and 7b), they differed in terms of metabolism and growth. The RD3-MolPack64 cells grew faster (T = 25.7±1.4 hours vs T = 32.7±2.9 SEM hours) (Fig. 7c and 7e) and after 4-7 days of culture their viability decreased increasing at the same time, the number of viable cells in suspension (Fig. 7d and 7f). Altogether, these findings indicate that RD3-MolPack system is preferable for fixed bed and hollow fiber based bioreactor, while RD2-MolPack for suspension bioreactor platform.

### Medium scale growth of RD3-MolPack24 cells and LV concentration

Once established that RD3-MolPack24 cells were the best candidate for adherent growth, we optimized their culture conditions and LV concentration towards medium-large scale production. We observed almost 4-fold increment of the titer (from 4.0 × 10⁵ to 1.5 × 10⁶ TU/ml) after optimization of cell culture and harvest conditions and a slightly higher titer when the clarified supernatants were centrifuged at low rather than high speed (Table 3). We then inoculated 1.7 × 10⁷ cells in the multilayer HYPERFlask™. From the sixth day, when the cells reached the optimal concentration for supernatant production (i.e. 2.2-2.3 × 10⁵ cells/cm² equal to 4 × 10⁸ cells/HYPERFlask™), we collected in six independent harvests, almost 3L of supernatant containing 2.4 × 10⁹ total TU with a mean infectivity of 7.8 × 10³±8.1× 10² TU/ng p24Gag (Table S2). Taken together these results are encouraging for future set up of large scale manufacturing either in multi-stack vessels or in bioreactor.

**Table 3: RD3-MolPack24 production in HYPERFlask™**

| **Harvest time** | **Titer (TU/ml)^{a}** | **p24Gag (ng/ml)^{a}** | **Infectivity (TU/ng)^{a}** | **Harvest Volum** | **Total TU^{a}** |
|---|---|---|---|---|---|
| 72 | 4.9 × 10⁵ | 065.5 | 7.4 × 10³ | 500 | 2.4 × 10⁸ |
| 24 | 4.6 × 10⁵ | 044.5 | 1.0 × 10⁴ | 500 | 2.3 × 10⁸ |
| 24 | 5.8 × 10⁵ | 071.6 | 8.2 × 10³ | 500 | 2.9 × 10⁸ |
| 24 | 1.1 × 10⁶ | 134.8 | 8.5 × 10³ | 500 | 5.7 × 10⁸ |
| 24 | 1.3 × 10⁶ | 148.2 | 8.6 × 10³ | 500 | 6.4 × 10⁸ |
| 72 | 1.1 × 10⁶ | 260.6 | 4.1 × 10³ | 400 | 4.3 × 10⁸ |
| | | | **Total** | 2,900 | 2.4 × 10⁹ |

| | | | | | |
|---|---|---|---|---|---|
| ^{a}Values are representative of one of two experiments | | | | | |

### Cells

Human embryo kidney 293T (HEK-293T) cells and its derivatives were propagated in Iscove's Modified Dulbecco's Modified Eagle Medium (IMDM) supplemented with 10% FCS and Penicillin-Streptomycin-Glutamine (PSG) (Lonza, Basel, Switzerland). CEM A3.01 T cells were grown in RPMI 1640 supplemented with 10% FCS and PSG. Human peripheral blood mononuclear cells (PBMC) were isolated from healthy donors after centrifugation on a Ficoll-Hypaque gradient (Lymphoprep; Stemcell Technologies, Vancouver, Canada) and then frozen in aliquots. PBMC were subsequently thawed and cultured in RPMI 1640 supplemented with 10% FCS, PSG and IL-7 and IL-15 (5 ng/ml, each) (Miltenyi Biotec, Bergisch Gladbach, Germany) after pre-activation with CD3/CD28 Dynabeads (LifeTechnologies Italia, Monza, Italy).

### Transient and continuous lentiviral vector (LV) production, concentration, cell transduction and titer calculation

LVs were obtained by transient co-transfection of HEK-293T cells with the following plasmids: the packaging constructs pCMV-ΔR8.9 (3rd-gen.), the pMD.G plasmid encoding the VSV-G envelope or the SIN-RD114-TR plasmids encoding the RD114-TR envelope, and the 3rd-gen. (SIN-GFP) transfer vector (TV). Supernatants were harvested 48 hours after transfection.

Continuous LV production was routinely obtained by cultivating RD-MolPack cells in different cell culture devices: standard flasks, a small disposable bioreactor and Hyperflasks. To evaluate the productivity of the RD-MolPack cells, T75 flasks were inoculated with 2.5 × 10⁶/cm2 in 0.5 ml/cm2 IMDM supplemented with 10% FBS and PSG and after 24 hours supernatant was harvested. RD-MolPack LVs were also produced by inoculating 2.5 × 10⁷ RD-MolPack cells in the CELLine AD 1000 bioreactor (INTEGRA Biosciences AG, CH) and replacing the cell compartment medium (15-ml IMDM + 10% FCS) every 48 hours, corresponding to a single harvest, whereas the medium compartment (1000-ml IMDM + 1% FCS) weekly for up to 3 months. In selected experiments, RD-MolPack cells were cultured in Corning® HYPERFlaskTM (Corning Inc. Life Science, Lowell, MA) by inoculating 1.7 × 10⁷ cells/flask and collecting the supernatant after 6 days, when the total number of cells reached approximately 4 × 10⁸. In all production systems, the harvested supernatant was clarified by 0.45-µm filtration and stored at - 80°C until use. When indicated, the supernatants were concentrated 100-fold by either low speed (3,761 × g at +4°C for 16 hours in a Multifuge 32-R centrifuge) or high speed centrifugation (50,000 × g at +4°C for 2 hours in a Beckman L-80 ultracentrifuge). The viral pellets were resuspended in IMDM medium supplemented with 10% FBS and frozen at -80°C for deferred use. Transduction of CEM A3.01 cell line and activated T lymphocytes was carried out by one cycle of spinoculation at 1,024 × g for 2 hours at 37°C in the presence of polybrene (8 µg/ml) (Sigma-Aldrich, St Louis, MO); transduction efficiency was monitored by flow cytometry analysis (FACS Canto II Instrument, BD Bioscience, San Jose, CA) of GFP (SIN-GFP) by the DIVA software (BD Bioscience). LV titer was calculated on CEM A3.01 cells using the 5-25% range of transduction efficiency as previously described [Porcellini et al.2010]. Physical particles (pp) production was estimated by measuring the p24Gag released in the supernatants by the Alliance HIV-1 p24 Antigen ELISA kit (Perkin Elmer, Inc. Waltham, MA) following manufacturer's instructions. It is assumed that 1 ng of p24Gag corresponds to 1 × 10⁷ pp.

### Western blot assay

Cellular and viral proteins, the latter derived from isolated cell-free VLPs or LVs, were prepared as previously described and then separated by SDS-PAGE on 4-15% Mini-PROTEAN® TGX™ precast gels (Biorad, Hercules, CA, USA). The primary antibodies used were the following: anti-HIV serum, obtained from an AIDS patient, and kindly donated by G. Poli (OSR, Milano, Italy) diluted 1:1,000; the anti-TM RD114-TR rabbit serum, kindly provided by F.L. Cosset (INSERM, France) diluted 1:1,000; anti-Rev mouse antibody, from Santa Cruz Biotechnology (sc-69730), diluted 1:500; anti-Tat mouse antibody, obtained from the NIH AIDS Research and Reference Reagent Program (USA), diluted 1:500; anti-actin rabbit antibody, (A 2066) (Sigma-Aldrich), diluted 1:2,500. The secondary HRP-linked antibodies used were: anti-human (NA933V) and anti-rabbit (NA934V) (GE Healthcare, Europe GmbH) diluted 1:5,000; anti-mouse (A2066) (Sigma-Aldrich) diluted 1:10,000. For the chemiluminescence reaction we used the ECL-Western Blotting Detection Reagents (RPMN2106) (GE Healthcare).

### RD3-MolPack24 and RD2-MolPack64 cell metabolism

RD3-MolPack24 and RD2-MolPack64 cells were seeded at 1.5 × 104 cells/cm2 density in T25 flasks. Cells were counted daily for a week and at each time point, supernatants were harvested to measure glucose consumption and lactate production by the YSI 2700 SELECT Biochemistry Analyzer (YSI, Yellow Springs, OH) and the functional and physical titers as described above.

## Claims

1. A system for the stable integration of a self-inactivating (SIN) lentiviral transfer vector into a packaging cell line wherein such system consists of a DNA fragment comprising, starting from the 5' end:
i. The Inverted Terminal Repeat of the Adeno Associated Virus (AAV ITR)
ii. A SIN lentiviral transfer vector
iii. A constitutive promoter that regulates the expression of an antibiotic resistance gene
iv. An antibiotic resistance gene
v. The Inverted Terminal Repeat of the Adeno Associated Virus (AAV ITR)

2. A system according to claim 1 wherein the SIN lentiviral transfer vector comprises at position 5' a lentiviral LTR in which the U3 segment has been substituted by a promoter selected from CMV, CMV IE, PGK, SV40, eF1α SFFV and RSV and at position 3', a deletion of the enhancer and promoter sequences of the lentiviral LTR.

3. A system according to claim 2 wherein the 5' U3 segment has been substituted by a CMV IE promoter.

4. A system according to anyone of claims 1 to 3 wherein the antibiotic resistance gene is selected from hygromycin, kanamycin, neomycin, bleomycin, phleomycin or Zeocin® resistance gene.

5. A system according to claim 4 wherein the antibiotic resistance gene is Zeocin® resistance gene

6. A system according to anyone of claims 1 to 5 wherein the promoter that regulates the expression of the resistance gene is selected from a CMV, CMV IE, PGK, SV40, eF1α, SFFV and RSV promoter.

7. A system according to claim 7 wherein the promoter that regulates the expression of the resistance gene is a SV40 promoter.

8. A method to obtain a producer cell line for self-inactivating (SIN) lentiviral vectors comprising:
i. Preparing a DNA fragment comprising, starting from the 5' end: the Inverted Terminal Repeat of the Adeno Associated Virus (AAV ITR), a SIN lentiviral transfer vector, a constitutive promoter that regulates the expression of an antibiotic resistance gene, an antibiotic resistance gene and the Inverted Terminal Repeat of the Adeno Associated Virus (AAV ITR)
ii. Transfecting a packaging cell line for lentiviral vector with such DNA fragment
iii. Selecting the producer cell line by culturing the cells in the presence of the antibiotic

9. A method according to claim 8 wherein the SIN lentiviral transfer vector comprises at position 5' a lentiviral LTR in which the U3 segment has been substituted by a promoter selected from CMV, CMV IE, PGK, SV40, eF1α SFFV and RSV and, at position 3', a deletion of the enhancer and promoter sequences of the lentiviral LTR.

10. A method according to claim 9 wherein the 5' U3 segment has been substituted by a CMV IE promoter.

11. A method according to anyone of claims 8 to 10 wherein the antibiotic resistance gene is selected from hygromycin, kanamycin, neomycin, bleomycin, phleomycin or Zeocin® resistance gene.

12. A method according to claim 11 wherein the antibiotic resistance gene employed in the system is Zeocin® resistance gene.

13. A method according to anyone of claims 8 to 12 wherein the promoter that regulates the expression of the antibiotic resistance gene is selected from a CMV, CMV IE, PGK, SV40, eF1α, SFFV and RSV, promoter.

14. A method according to claim 13 wherein the promoter that regulates the expression of the resistance gene is a SV40 promoter.

15. A method according to anyone of claims 8 to 14 wherein the packaging cell line is a stable packaging cell line obtained from an host cell line containing into its genome lentiviral gag/pol, lentiviral rev and an envelope protein.

16. A method according to anyone of claims 8 to 15 wherein the packaging cell line further, contains lentiviral tat stably integrated into the genome.

17. A method according to claims 15 or 16 wherein the host cell line is a human cell line selected from HEK293, HEK293-T, HEK293-SF, TE671, HT1080 or HeLa.

18. A method according to anyone of claims 15 to 17 wherein the env gene is selected from VSV-G env, MLV 4070 env, RD114 env, chimeric envelope protein RD114-TR, chimeric envelope protein RD114pro, baculovirus GP64 env or GALV env or derivatives thereof.

19. A producer cell line obtainable by the method of any one of claims 8 to 18.

20. A producer cell line containing into its genome at least one copy of a DNA fragment comprising of starting from the 5' end:
i. The Inverted Terminal Repeat of the Adeno Associated Virus (AAV ITR)
ii. A self-inactivating (SIN) lentiviral transfer vector
iii. A constitutive promoter that regulates the expression of an antibiotic resistance gene
iv. An antibiotic resistance gene
v. The Inverted Terminal Repeat of the Adeno Associated Virus (AAV ITR).

21. A process for producing self-inactivating (SIN) lentiviral vectors comprising culturing a producer cell line containing into its genome at least one copy of a DNA fragment comprising starting from the 5' end:
i. The Inverted Terminal Repeat of the Adeno Associated Virus (AAV ITR)
ii. A SIN lentiviral transfer vector
iii. A constitutive promoter that regulates the expression of an antibiotic resistance gene
iv. An antibiotic resistance gene
v. The Inverted Terminal Repeat of the Adeno Associated Virus (AAV ITR).

## Patentansprüche

1. System zur stabilen Integration eines selbstinaktivierenden (SIN) lentiviralen Transfervektors in eine Verpackungszelllinie, wobei ein solches System aus einem DNA-Fragment besteht, das ausgehend vom 5'-Ende umfasst:
i. die invertierte terminale Wiederholung des Adeno-assoziierten Virus (AAV-ITR),
ii. einen lentiviralen SIN-Transfervektor,
iii. einen konstitutiven Promotor, der die Expression eines Antibiotikaresistenzgens reguliert,
iv. ein Antibiotikaresistenzgen,
v. die invertierte terminale Wiederholung des Adeno-assoziierten Virus (AAV-ITR).

2. System gemäß Anspruch 1, wobei der lentivirale SIN-Transfervektor an Position 5' eine lentivirale LTR umfasst, bei der das U3-Segment durch einen Promotor ersetzt wurde, der aus CMV, CMV IE, PGK, SV40, eF1α, SFFV und RSV ausgewählt ist, und an Position 3' eine Deletion der Enhancer- und Promotorsequenzen der lentiviralen LTR aufweist.

3. System gemäß Anspruch 2, wobei das 5'-U3-Segment durch einen CMV-IE-Promotor ersetzt wurde.

4. System gemäß einem der Ansprüche 1 bis 3, wobei das Antibiotikaresistenzgen ausgewählt ist aus dem Hygromycin-, dem Kanamycin-, dem Neomycin-, dem Bleomycin-, dem Phleomycin- oder Zeocin®-Resistenzgen.

5. System gemäß Anspruch 4, wobei das Antibiotikaresistenzgen das Zeocin®-Resistenzgen ist.

6. System gemäß einem der Ansprüche 1 bis 5, wobei der Promotor, der die Expression des Resistenzgens reguliert, aus einem CMV-, einem CMV-IE-, einem PGK-, einem SV40-, einem eF1a-, einem SFFV- und einem RSV-Promotor ausgewählt ist.

7. System gemäß Anspruch 7, wobei der Promotor, der die Expression des Resistenzgens reguliert, ein SV40-Promotor ist.

8. Verfahren zum Erhalten einer Produzentenzelllinie für selbstinaktivierende (SIN) lentivirale Vektoren, umfassend:
i. Herstellen eines DNA-Fragments, das ausgehend vom 5'-Ende umfasst: die invertierte terminale Wiederholung des Adeno-assoziierten Virus (AAV-ITR), einen lentiviralen SIN-Transfervektor, einen konstitutiven Promotor, der die Expression eines Antibiotikaresistenzgens reguliert, ein Antibiotikaresistenzgen und die invertierte terminale Wiederholung des Adeno-assoziierten Virus (AAV-ITR),
ii. Transfizieren einer Verpackungszelllinie für einen lentiviralen Vektor mit einem solchen DNA-Fragment,
iii. Auswählen der Produzentenzelllinie durch Kultivieren der Zellen in Gegenwart des Antibiotikums.

9. Verfahren gemäß Anspruch 8, wobei der lentivirale SIN-Transfervektor an Position 5' eine lentivirale LTR umfasst, bei der das U3-Segment durch einen Promotor ersetzt wurde, der aus CMV, CMV IE, PGK, SV40, eF1α, SFFV und RSV ausgewählt ist, und an Position 3' eine Deletion der Enhancer- und Promotorsequenzen der lentiviralen LTR aufweist.

10. Verfahren gemäß Anspruch 9, wobei das 5'-U3-Segment durch einen CMV-IE-Promotor ersetzt wurde.

11. Verfahren gemäß einem der Ansprüche 8 bis 10, wobei das Antibiotikaresistenzgen ausgewählt ist aus dem Hygromycin-, dem Kanamycin-, dem Neomycin-, dem Bleomycin-, dem Phleomycin- oder Zeocin®-Resistenzgen.

12. Verfahren gemäß Anspruch 11, wobei das in dem System verwendete Antibiotikaresistenzgen das Zeocin®-Resistenzgen ist.

13. Verfahren gemäß einem der Ansprüche 8 bis 12, wobei der Promotor, der die Expression des Antibiotikaresistenzgens reguliert, aus einem CMV-, einem CMV-IE-, einem PGK-, einem SV40-, einem eF1a-, einem SFFV- und einem RSV-Promotor ausgewählt ist.

14. Verfahren gemäß Anspruch 13, wobei der Promotor, der die Expression des Resistenzgens reguliert, ein SV40-Promotor ist.

15. Verfahren gemäß einem der Ansprüche 8 bis 14, wobei die Verpackungszelllinie eine stabile Verpackungszelllinie ist, die aus einer Wirtszelllinie erhalten wird, die in ihrem Genom lentivirales gag/pol, lentivirales rev und ein Hüllprotein enthält.

16. Verfahren gemäß einem der Ansprüche 8 bis 15, wobei die Verpackungszelllinie ferner lentivirales tat enthält, das stabil in das Genom integriert ist.

17. Verfahren gemäß Anspruch 15 oder 16, wobei die Wirtszelllinie eine menschliche Zelllinie ist, ausgewählt aus HEK293, HEK293-T, HEK293-SF, TE671, HT1080 oder HeLa.

18. Verfahren gemäß einem der Ansprüche 15 bis 17, wobei das env-Gen ausgewählt ist aus VSV-G env, MLV 4070 env, RD114 env, chimärem Hüllprotein RD114-TR, chimärem Hüllprotein RD114pro, Baculovirus GP64 env oder GALV env oder Derivaten davon.

19. Produzentenzelllinie, erhältlich nach dem Verfahren nach einem der Ansprüche 8 bis 18.

20. Produzentenzelllinie, die in ihrem Genom mindestens eine Kopie eines DNA-Fragments enthält, das beginnend am 5'-Ende umfasst:
i. die invertierte terminale Wiederholung des Adeno-assoziierten Virus (AAV-ITR),
ii. einen selbstinaktivierenden (SIN) lentiviralen Transfervektor,
iii. einen konstitutiven Promotor, der die Expression eines Antibiotikaresistenzgens reguliert,
iv. ein Antibiotikaresistenzgen,
v. die invertierte terminale Wiederholung des Adeno-assoziierten Virus (AAV-ITR).

21. Verfahren zur Herstellung von selbstinaktivierenden (SIN) lentiviralen Vektoren, umfassend das Kultivieren einer Produzentenzelllinie, die in ihrem Genom mindestens eine Kopie eines DNA-Fragments enthält, das beginnend am 5'-Ende umfasst:
i. die invertierte terminale Wiederholung des Adeno-assoziierten Virus (AAV-ITR),
ii. einen lentiviralen SIN-Transfervektor,
iii. einen konstitutiven Promotor, der die Expression eines Antibiotikaresistenzgens reguliert,
iv. ein Antibiotikaresistenzgen,
v. die invertierte terminale Wiederholung des Adeno-assoziierten Virus (AAV-ITR).

## Revendications

1. Système destiné à l'intégration stable d'un vecteur de transfert lentiviral à auto-inactivation (SIN) dans une lignée cellulaire d'encapsidation, dans lequel un tel système se compose d'un fragment d'ADN comprenant, à partir de l'extrémité 5' :
i. La répétition terminale inversée du virus adéno associé (AAV ITR)
ii. Un vecteur de transfert lentiviral à SIN
iii. Un promoteur constitutif qui régule l'expression d'un gène de résistance aux antibiotiques
iv. Un gène de résistance aux antibiotiques
v. La répétition terminale inversée du virus adéno associé (AAV ITR).

2. Système selon la revendication 1 dans lequel le vecteur de transfert lentiviral à SIN comprend au niveau de la position 5' une longue répétition terminale (LTR) lentivirale dans laquelle le segment U3 a été substitué par un promoteur sélectionné parmi CMV, CMV IE, PGK, SV40, eF1α SFFV et RSV et à la position 3', une suppression des séquences amplificatrices et promotrices de la LTR lentivirale.

3. Système selon la revendication 2 dans lequel le segment U3 5' a été substitué par un promoteur de CMV IE.

4. Système selon l'une quelconque des revendications 1 à 3, dans lequel le gène de résistance aux antibiotiques est choisi parmi le gène de résistance hygromycine, kanamycine, néomycine, bléomycine, phléomycine ou Zeocin®.

5. Système selon la revendication 4, dans lequel le gène de résistance aux antibiotiques est le gène de résistance Zeocin®.

6. Système selon l'une quelconque des revendications 1 à 5, dans lequel le promoteur qui régule l'expression du gène de résistance est choisi parmi un promoteur CMV, CMV IE, PGK, SV40, eF1α, SFFV et RSV.

7. Système selon la revendication 7, dans lequel le promoteur qui régule l'expression du gène de résistance est un promoteur SV40.

8. Procédé d'obtention d'une lignée cellulaire productrice destinée aux vecteurs lentiviraux à auto-inactivation (SIN) comprenant :
i. La préparation d'un fragment d'ADN comprenant, à partir de l'extrémité 5' : la répétition terminale inversée du virus adéno associé (AAV ITR), un vecteur de transfert lentiviral à SIN, un promoteur constitutif qui régule l'expression d'un gène de résistance aux antibiotiques, un gène de résistance aux antibiotiques et la répétition terminale inversée du virus adéno associé (AAV ITR)
ii. La transfection d'une lignée cellulaire d'encapsidation destinée à un vecteur lentiviral avec un tel fragment d'ADN
iii. La sélection de la lignée cellulaire productrice en cultivant les cellules en présence de l'antibiotique.

9. Procédé selon la revendication 8 dans lequel le vecteur de transfert lentiviral à SIN comprend au niveau de la position 5' une longue répétition terminale (LTR) lentivirale dans laquelle le segment U3 a été substitué par un promoteur sélectionné parmi CMV, CMV IE, PGK, SV40, eF1α SFFV et RSV et à la position 3', une suppression des séquences amplificatrices et promotrices de la LTR lentivirale.

10. Procédé selon la revendication 9 dans lequel le segment U3 5' a été substitué par un promoteur de CMV IE.

11. Procédé selon l'une quelconque des revendications 8 à 10, dans lequel le gène de résistance aux antibiotiques est choisi parmi le gène de résistance hygromycine hygromycine, kanamycine, néomycine, bléomycine, phléomycine ou Zeocin®.

12. Procédé selon la revendication 11, dans lequel le gène de résistance aux antibiotiques employé dans le système est le gène de résistance Zeocin®.

13. Procédé selon l'une quelconque des revendications 8 à 12, dans lequel le promoteur qui régule l'expression du gène de résistance aux antibiotiques est choisi parmi un promoteur CMV, CMV IE, PGK, SV40, eF1α, SFFV et RSV.

14. Procédé selon la revendication 13, dans lequel le promoteur qui régule l'expression du gène de résistance est un promoteur SV40.

15. Procédé selon n'importe qui des revendications 8 à 14, dans lequel la lignée cellulaire d'encapsidation est une lignée cellulaire d'encapsidation stable obtenue à partir d'une lignée cellulaire hôte contenant dans son génome le lentiviral gag/pol, le lentiviral rev et une protéine d'enveloppe.

16. Procédé selon l'une quelconque des revendications 8 à 15, dans lequel la lignée cellulaire d'encapsidation contient en outre le lentiviral tat intégré de manière stable dans le génome.

17. Procédé selon la revendication 15 ou la revendication 16, dans lequel la lignée cellulaire hôte est une lignée cellulaire humaine sélectionnée à partir de HEK293, HEK293-T, HEK293-SF, TE671, HT1080 ou HeLa.

18. Procédé selon l'une quelconque des revendications 15 à 17, dans lequel le gène env est choisi parmi VSV-G env, MLV 4070 env, RD114 env, la protéine d'enveloppe chimérique RD114-TR, la protéine d'enveloppe chimérique RD114pro, le baculovirus GP64 env ou GALV env ou ses dérivés.

19. Lignée cellulaire productrice pouvant être obtenue par le procédé selon l'une quelconque des revendications 8 à 18.

20. Lignée cellulaire productrice contenant dans son génome au moins une copie d'un fragment d'ADN comprenant, à partir de l'extrémité 5' :
i. La répétition terminale inversée du virus adéno associé (AAV ITR)
ii. Un vecteur de transfert lentiviral à auto-inactivation (SIN)
iii. Un promoteur constitutif qui régule l'expression d'un gène de résistance aux antibiotiques
iv. Un gène de résistance aux antibiotiques
v. La répétition terminale inversée du virus adéno associé (AAV ITR).

21. Processus destiné à la production de vecteurs lentiviraux à auto-inactivation (SIN) comprenant la culture d'une lignée cellulaire productrice contenant dans son génome au moins une copie d'un fragment d'ADN comprenant, à partir de l'extrémité 5' :
i. La répétition terminale inversée du virus adéno associé (AAV ITR)
ii. Un vecteur de transfert lentiviral à SIN
iii. Un promoteur constitutif qui régule l'expression d'un gène de résistance aux antibiotiques
iv. Un gène de résistance aux antibiotiques
v. La répétition terminale inversée du virus adéno associé (AAV ITR).
